**Europäisches Patentamt**

**European Patent Office**

**Office européen des brevets**

(11) Publication number : **0 191 217 B2**

(12) **NEW EUROPEAN PATENT SPECIFICATION**

(45) Date of publication of the new patent specification : **19.02.92 Bulletin 92/08**

(51) Int. Cl.$^5$ : **C12P 7/64, C11C 3/02**

(21) Application number : **85305796.6**

(22) Date of filing : **14.08.85**

(54) Process for producing glycerides in the presence of lipases.

(30) Priority : **06.02.85 JP 21546/85**

(43) Date of publication of application :
**20.08.86 Bulletin 86/34**

(45) Publication of the grant of the patent :
**16.03.88 Bulletin 88/11**

(45) Mention of the opposition decision :
**19.02.92 Bulletin 92/08**

(84) Designated Contracting States :
**DE FR GB IT**

(56) References cited :
CHEMICAL ABSGTRACTS, vol. 101, no. 15,
October 1984, page 573, no. 128870e, Colum-
bus, Ohio, US; & JP - A - 59 118 094 (NIPPON
OILS AND FATS CO., LTD) 07-07-1984
JOURNAL OF THE AMERICAN OIL CHEMISTS
SOCIETY, vol. 61, no. 2, February 1984, pages
290-297, Champaign, Illinois, US; S.L. NEIDEL-
MAN et al.: "Biotechnology and oleochem-
icals: Changing patterns"

(56) References cited :
AGRICULTURAL AND BIOLOGICAL CHEMIS-
TRY, vol. 45, no. 1, 1981, pages 185-189, Tokyo,
JP; S. OKUMURA et al.: "The effect of reverse
action on triglyceride hydrolysis by lipase"
JOURNAL OF CHROMOTOGRAPHY, vol. 45,
1969, pages 473-475, Amsterdam, NL; P.S.
STEYN: "The separation and detection of sev-
eral mycotoxins by thin-layer
chromatography"
Biochim/Biophys. Acta 489 (1977), page 415 -
422
J. Biochem 87 (1980) pages 205 - 211

(73) Proprietor : **AMANO PHARMACEUTICAL CO.,
LTD.**
2/7, Nishiki 1-chome, Naka-ku
Nagoya-shi Aichi-ken (JP)

(72) Inventor : **Yamaguchi, Shotaro**
6, Kitaguchi Ohaza Kunotsubo
Nishiharu-machi
Nishi Kasugai-gun Aichi-ken (JP)
Inventor : **Mase, Tamio**
49-2, Machiyago Nishi Chiaki-cho
Ichinomiya-shi Aichi-ken (JP)
Inventor : **Asada, Satoru**
1311, Kawai-cho
Iwakura-shi Aichi-ken (JP)

(74) Representative : **West, Alan Harry et al**
R.G.C. Jenkins & Co. 26 Caxton Street
London SW1H 0RJ (GB)

EP 0 191 217 B2

## Description

This invention relates to a process for producing glycerides using an enzyme. More particularly, the present invention relates in one aspect to a process for synthesizing glycerides by reacting a fatty acid or a fatty acid ester with glycerol in the presence of a monoglyceride lipase and/or a diglyceride lipase to produce glycerides containing little or no triglyceride and consisting substantially of monoglyceride and diglyceride; and in another aspect to a process for synthesizing glycerides by reacting a fatty acid or a fatty acid ester with glycerol in the presence of such a lipase to produce glycerides completely or substantially free from triglyceride and diglyceride and consisting substantially of monoglyceride. Monoglycerides and diglycerides are useful as emulsifiers and antimicrobial agents.

Lipases are enzymes which hydrolyse fats and oils and esters of fatty acids. However, it is known that under certain conditions, lipases cause esterification which is the reverse of hydrolysis; thereby glycerides or esters can be synthesized from fatty acid and glycerol or from fatty acid and alcohol (J. Gen. Appl. Microbiol., 10, 13-22, 1964; Proc. IV IFS: Ferment. Technol. Today, 315-320, 1972, issued by Society of Fermentation Technology Japan; Bull. Tech/Gattefosse Rep., 76, 34-36, 1983; J. Am. Oil Chem. Soc., 61, 191-195, 1984; Japanese Patent Publication No. 7754/1976, Japanese Patent Publication No. 23535/1982, Japanese Patent Laid-Open No. 118094/1984, Japanese Patent Laid-Open No. 118095/1984, etc). The lipases used in this prior art and the most popular lipases are triacylglycerol lipases which are also called triglyceride lipases. These lipases have a specificity not only to triglycerides but also to diglycerides and monoglycerides, although the specificities to each glyceride are different. Glycerides synthesized by esterification using the action of these triglyceride lipases are usually a mixture of triglyceride, diglyceride, and monoglyceride and, even when these glycerides consist substantially of monoglyceride and diglyceride, the content of monoglyceride in the glyceride mixture is low.

A paper by Tsujisaka et al in Biochimica et Biophysica Acta 489 (1977) at pages 415-422 compares the actions of four lipases including crude A-lipase derived from Penicillium cyclopium ATCC 34613, in the synthesis of glycerides. From the data presented in that paper, it is evident that A-lipase exhibits only a narrow spectrum of activity in that it functions only on lauric and oleic acids and then only in the presence of proteins or buffer to maintain pH.

A later paper by the same authors in J. Biochem. 87 (1980) at pages 205-211 describes the purification and properties of a further lipase derived from Penicillium cyclopium ATCC 34613, referred to by the authors as enzyme-II. That product is shown to be extremely active for the rapid and complete hydrolysis of mono- and diglycerides.

Monoglycerides and diglycerides are in wide use as emulsifiers for foods, cosmetics, medicines, etc. It is known that, in their ability to act as an emulsifier, monoglycerides are far superior to diglycerides and the presence of triglycerides is not desirable. Particular types of monoglycerides such as monolaurin, monocaprin and the like are in use as antimicrobial agents or antiseptic agents, e.g. for foods. In general, glycerides used as emulsifiers are required to contain at least about 90 mole % of monoglycerides. Hence, in the conventional production of such glycerides, it has been necessary to subject a glyceride mixture to molecular distillation or the like to enhance the content of monoglycerides.

The present invention seeks to provide a process for producing glycerides consisting substantially of monoglyceride and diglyceride, useful as emulsifiers and antimicrobial agents, as well as to provide a process for producing glycerides consisting substantially of monoglyceride.

According to one aspect of the invention, there is provided monoglycerides and diglycerides substantially free from triglycerides which comprises reacting glycerol with a fatty acid or an ester of a fatty acid and agitating the reaction mixture in the presence of a monoglyceride lipase and/or a diglyceride lipase, characterised in that the lipase is enzyme-II derived from Penicillium cyclopium.

According to another aspect of the invention, there is provided a process for producing monoglycerides substantially free from triglycerides and diglycerides which comprises reacting glycerol with a fatty acid or an ester of a fatty acid and agitating the reaction mixture in the presence of a monoglyceride lipase and/or a diglyceride lipase, characterised in that the lipase is enzyme-II derived from Penicillium cyclopium, and that the reaction is carried out for 1 to 50 hours at an initial glycerol to raw material fatty acid or ester mole ratio from 0.2-50:1 in the presence of from 2 to 5,000 units of the lipase per mole of raw material fatty acid or ester thereof.

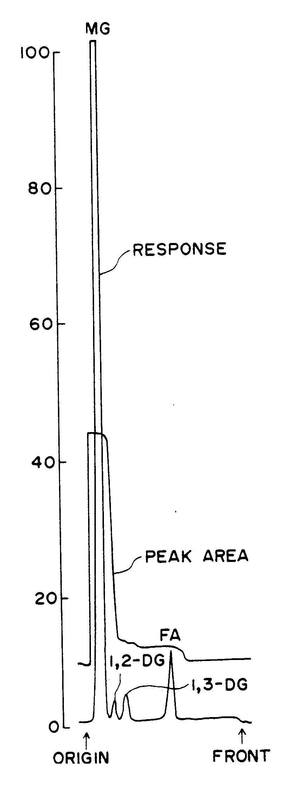

The accompanying drawing illustrates a glyceride composition prepared according to the present process, analyzed using thin-layer chromatography and a hydrogen flame ionization detector. In the drawing, MG is monoglyceride; 1,2-DG is 1,2-diglyceride; 1,3-DG is 1,3-diglyceride; and FA is fatty acid.

According to the present invention, a fatty acid or fatty acid ester is reacted with glycerol in the presence of a monoglyceride lipase and/or a diglyceride lipase under appropriate conditions, whereby glycerides are synthesized completely or substantially free from triglyceride or substantially free from triglyceride and diglyceride.

EP 0 191 217 B2

The lipase used in the present invention is an enzyme which can hydrolyse a monoglyceride wherein one of the three hydroxyl groups of glycerol is esterified with a fatty acid and/or a diglyceride wherein the two hydroxyl groups at the 1,2- or 1,3- or 2,3-positions of glycerol are esterified with a fatty acid, but has little or no effect on triglycerides wherein all three hydroxyl groups of glycerol are esterified by fatty acid.

The lipase used in the invention is enzyme-II derived from Penicillium cyclopium an preferably derived from the Penicillium cyclopium deposited with the American Type Culture Collection under the Accession Number ATCC 34613. This strain is listed in the ATCC catalogue and is readily available.

It is know that, besides producing a triglyceride lipase, Penicillium cyclopium produces a lipase having a specificity for monoglycerides and diglycerides (J. Biochem., 87, 205-211, 1980). However, the present inventors do not know any literature suggesting that said lipase is usable for the purpose of the present invention.

To prepare a lipase for use in the present invention, a strain of Penicillium cyclopium capable of producing a lipase having a specificity for monoglycerides and/or diglycerides is grown in a medium used in ordinary cultivation of microorganisms and the lipase accumulated in the cultivation mixture is collected. Purification of the lipase collected from the cultivation mixture can be conducted using known purification means; however, for the purpose of the present invention, there is no need to purify the lipase. Only in the case where the crude lipase contains a triglyceride lipase must the triglyceride lipase be removed. Although a cultivation mixture obtained with the preferred Penicillium cyclopium ATCC 34613 contains a triglyceride lipase, this lipase can be separated and removed by subjecting a crude enzyme solution prepared form the cultivation mixture to chromatography, particularly preferably chromatography using DEAE-Sepharose CL-6B.

In the present invention, the preferred fatty acids are those having from 4 to 22 carbon atoms; they may be saturated or unsaturated and may have straight or branched chains. The preferred esters are the methyl, ethyl, propyl, butyl, benzyl, amyl and vinyl esters of such fatty acids. Also useful are hydrolyzates of fats and oils obtained by physicochemical means such as an autoclave process, the Twitchell method, saponification and the like or by enzymatic means using a triglyceride lipase. Especially preferred fatty acids are, for example, stearic acid, oleic acid, linoleic acid, linolenic acid, palmitic acid, myristic acid, lauric acid, capric acid, caprylic acid, caproic acid and isostearic acid, and especially preferred esters are, for example, vinyl stearate, vinyl palmitate, vinyl laurate and vinyl caprate.

According to the present invention, the proportions of product monoglycerides and diglycerides can be varied widely by selecting reaction conditions appropriately. Thus, the ratio of monoglycerides in the product glycerides can vary from about 50% (molar ratio) to substantially 100%.

In order to synthesize a mixture of monoglycerides and diglycerides, it is preferred that the reaction time is 1 hour to 5 days, the amount of lipase is 2 to 10,000 units per mole of raw material fatty acid or ester thereof and the mole ratio of glycerol to raw material fatty acid or ester thereof is 0.2-200:1.

In order to synthesize substantially only monoglycerides, it is necessary that the reaction time is 1 to 50 hours, the amount of lipase is 2 to 5, 000 units per mole of raw material fatty acid or ester thereof and the initial glycerol to raw material fatty acid or ester mole ratios 0. 2-50: 1.

In both cases, i.e. for producing a mixture of monoglycerides and diglycerides and for producing substantially only monoglycerides, it is preferred that the reaction temperature is from 20 to 55°C; that the water content in the reaction mixture is 30% by weight or less; and that the pH is maintained within the effective range for the lipase.

The resulting glyceride product can be separated from the reaction mixture by extraction with an organic solvent such as petroleum ether or the like, followed by vacuum distillation or alkaline refining to remove fatty acids. Unreacted raw material can be used again as a raw material for glyceride synthesis. When the glycerides obtained contain a sufficiently high proportion of monoglyceride, the mixture per se can be utilised as an emulsifier or an antimicrobial agent. If the monoglyceride content is not sufficient, the glycerides can be utilised after being subjected to molecular distillation, for example, to enhance the monoglyceride content. As mentioned above, it is possible to synthesise glycerides consisting substantially of monoglyceride only; in this case, molecular distillation or the like is unnecessary.

In the accompanying drawing, there is shown a typical example of composition analysis for glycerides synthesized from oleic acid and glycerol according to the invention. After completion of the reaction between oleic acid and glycerol, the reaction mixture was subjected to extraction with petroleum ether and then the components in the resulting extract were analyzed employing thin-layer chromatography on silica gel using a hydrogen flame ionization detector (lactroscan TH-10 manufactured by Iatron Laboratories, Inc.). As is clear from the drawing, the components in the glyceride obtained are mostly a monoglyceride (MG, namely, monoolein); a 1,2-diglyceride (1,2-DG, namely, 1,2-diolein) and a 1,3-diglyceride (1,3-DG, namely, 1,3-diolein) are present in small amounts and no triglyceride (namely, triolein) was synthesized. In the drawing, FA implies unreacted oleic acid.

The present invention will be explained in detail below by referring to Experiments and Examples. Analysis

3

of glycerides synthesised as well as measurement of the proportion of a fatty acid or an ester thereof consumed in glyceride synthesis (hereinunder this proportion is referred to as consumption rate) were made as follows. Also, the definition of unit of lipase is given below.

Composition Analysis of Glycerides Synthesized

The reaction mixture was extracted with petroleum ether. The components in the resulting extract were subjected to a preparative silica gel thin-layer plate (PLK 5 manufactured by Whatman Co.). Development was conducted with petroleum ether:diethyl ether:acetic acid (80:30:1 by volume) and spots were detected with iodine vapor. Each spot containing one glyceride was recovered and subjected to quantitative determination of each particular glyceride using a reagent composition (Triglyceride G-test Wako, manufactured by Wako Pure Chem. Ind., Ltd.) for enzymatic triglyceride determination using a triglyceride lipase, glycerol kinase, glycerol-3-phosphate oxidase and peroxidase. The proportion of each molecule species was expressed as mole %.

Consumption Rate of Raw Material Fatty Acid or Ester thereof

The reaction mixture was extracted with petroleum ether and the components in the resulting extract were analyzed with the above mentioned thin-layer chromatography using hydrogen flame ionization detector. The consumption rate of raw material fatty acid or ester thereof was expressed as percentile proportion of the sum of the peak area of glycerides obtained to the total peak area of glycerides obtained and unreacted fatty acid or ester thereof.

Definition of Unit of Lipase

0.95 ml of 50 mM acetate buffer solution (pH 5.6) containing 2.5 mM p-nitrophenyllaurate and 2.0% Triton X-100 was mixed with 0.05 ml of an enzyme solution. Incubation was conducted for 15 min at 37°C and then 2.0 ml of acetone was added to quench the reaction. Thereafter, the amount of p-nitrophenol liberated in the reaction was determined from an absorbance at 410 nm. The amount of lipase required for liberating 1 $\mu$ mole p-nitrophenol per min in the above reaction was defined as 1 unit.

Experiment 1

Preparation of Lipase Having a Specificity for Monoglycerides and Diglycerides

Penicillium cyclopium ATCC 34613 was inoculated into a liquid medium (pH 6.0) containing 2% of rice bran and 1.5% of corn steep liquor and cultivation was conducted for 2 days at 26°C with stirring and aeration. From the resulting cultivation mixture mycelia was removed by filtration. The resulting filtrate was subjected to ultrafiltration for concentration. The resulting concentrate was subjected to chromatography using DEAE-Sepharose CL-6B manufactured by Pharmacia Co. to separate and remove a triglyceride lipase present and to obtain a purified lipase preparation. It was ascertained that this lipase preparation hydrolyzes monoglycerides and diglycerides but has little hydrolyzing activity on triglycerides.

In the following, this lipase preparation was used.

Experiment 2

Relation between Reaction Time and Composition of Glycerides Synthesized

1.77 g of oleic acid, 8.0 g of glycerol, 0.2 ml (5 units) of a solution containing a lipase from Penicillium cyclopium and 0.35 ml of water were mixed. The reaction was conducted at 40°C with stirring. At 8, 24, 48 and 96 hrs. after the start of the reaction, samples of reaction mixtures at these points of time were collected to conduct composition analysis of respective glycerides formed. As shown in Table 1, each glyceride mixture obtained consisted of only a monoglyceride and a diglyceride and no triglyceride was detected. The proportion of monoglyceride reached 70% or more, and the shorter the reaction time, the higher was the monoglyceride content.

## TABLE 1

| Reaction time (hr) | Monoglyceride (%) | Diglyceride (%) | Triglyceride (%) |
|---|---|---|---|
| 8 | 95.6 | 4.4 | 0 |
| 24 | 90.7 | 9.3 | 0 |
| 48 | 78.9 | 21.1 | 0 |
| 72 | 78.4 | 21.6 | 0 |
| 96 | 73.3 | 26.8 | 0 |

Experiment 3

Relation between Amount of Lipase Used and Composition of Glycerices synthesized

In Experiment 2, the amount of lipase used was varied widely and the reaction was conducted for 20 hours at 40°C. As shown in Table 2, the smaller the amount of lipase used, the higher was the content of monoglyceride synthesized.

## TABLE 2

| Amount of lipase used (unit) | Monoglyceride (%) | Diglyceride (%) | Triglyceride (%) |
|---|---|---|---|
| 1.25 | 97.5 | 2.5 | 0 |
| 5.0 | 93.7 | 6.4 | 0 |
| 15.0 | 78.0 | 22.0 | 0 |
| 30.0 | 65.6 | 34.4 | 0 |
| 50.0 | 54.4 | 45.6 | 0 |

Experiment 4

Relation between (a) Ratio of Raw Materials (Fatty Acid versus Glycerol) and (b) Composition of Glycerides Synthesized

In Experiment 2, the proportion of oleic acid and glycerol was varied widely and the reaction was conducted for 20 hours at 40°C. As shown in Table 3, the smaller the proportion of glycerol to fatty acid, the higher was the content of monoglyceride synthesized.

TABLE 3

| Oleic acid:<br>Glycerol<br>(g):(g)<br>(Molar ratio) | Monoglyceride<br>(%) | Diglyceride<br>(%) | Triglyceride<br>(%) |
|---|---|---|---|
| 0.95:9.0<br>(1:29.0) | 70.1 | 29.9 | 0 |
| 1.9:8.0<br>(1:12.9) | 86.5 | 13.5 | 0 |
| 3.8:6.0<br>(1:4.8) | 89.4 | 10.6 | 0 |
| 5.6:4.0<br>(1:2.2) | 95.1 | 4.9 | 0 |

Example 1

1.77 g of oleic acid, 8.0 g of glycerol, and 0.2 ml (5 units) of a solution containing a lipase from Penicillium cyclopium were mixed, and the reaction was conducted for 20 hours at 30°C. The consumption rate of oleic acid was 47%. The resulting glycerides were extracted with petroleum ether analyzed for composition. The proportion of monoglyceride in total glycerides was 98.4% and that of diglyceride was 1.6%. No triglyceride was detected.

Example 2

0.44 g of oleic acid, 9.5 g of glycerol, and 0.2 ml (5 units) of a solution containing a lipase from Penicillium cyclopium were mixed and the reaction was conducted for 20 hours at 40°C. The consumption rate of oleic acid was 75% and the glycerides synthesized consisted of 88.2% of monoglyceride, 11.8% of diglyceride and 0% of triglyceride.

Example 3

1.77 g of oleic acid, 8.0 g of glycerol, 0.05 ml (5.5 units) of a solution containing a lipase from Penicillium cyclopium and 0.05 ml of water were mixed, and the reaction was conducted for 20 hours at 30°C. The consumption rate of oleic acid was 23% and the glycerides synthesized consisted of 94.9% of monoglyceride, 5.1 % of diglyceride and 0% of triglyceride.

Example 4

1.77 g of lauric acid or capric acid, 8.0 g of glycerol, 0.2 ml (5 units) of a solution containing a lipase from Penicillium cyclopium, and 0.35 ml of water were mixed, and the reaction was conducted for 20 hours at 40°C. The consumption rate of fatty acid and the composition of glycerides synthesized are shown in Table 4.

TABLE 4

| Raw<br>material<br>fatty<br>acid | Consumption<br>rate of<br>fatty acid<br>(%) | Mono-<br>glyceride<br>(%) | Di-<br>glyceride<br>(%) | Tri-<br>glyceride<br>(%) |
|---|---|---|---|---|
| Lauric acid | 30 | 95.8 | 4.2 | 0 |
| Capric acid | 41 | 95.2 | 4.8 | 0 |

Example 5

1.76 g of linoleic acid or 1.75 g of linolenic acid or 1.78 g of isostearic acid, 8.0 g of glycerol, 0.2 ml (5 units) of a solution containing a lipase from Penicillium cyclopium, and 0.35 ml of 1 M McIlvaine buffer solution (pH 6.0) were mixed, and the reaction was conducted for 20 hours at 40°C. The consumption rate of fatty acid and the composition of glycerides synthesized are shown in Table 5.

TABLE 5

| Raw material fatty acid | Consumption rate of fatty acid (%) | Mono-glyceride (%) | Di-glyceride (%) | Tri-glyceride (%) |
|---|---|---|---|---|
| Linoleic acid | 59 | 91.1 | 8.9 | 0 |
| Linolenic acid | 47 | 91.9 | 8.1 | 0 |
| Isostearic acid | 22 | 95.8 | 4.2 | 0 |

Example 6

1.77 g of stearic acid, palmitic acid, myristic acid, caprylic acid or caproic acid, 8.0 g of glycerol, 0.2 ml (5 units) of a solution containing a lipase from Penicillium cyclopium, and 0.35 ml of water were mixed, and the reaction was conducted for each mixture for 20 hours at 40°C. Each glyceride mixture synthesized consisted mostly of a monoglyceride. In each glyceride mixture, a diglyceride was present only in a trace amount and no triglyceride was detected.

Example 7

1.0 g of oleic acid, 1.0 g of palmitic acid, 8.0 g of glycerol, 0.2 ml (5 units) of a solution containing a lipase from Penicillium cyclopium, and 0.35 ml of water were mixed, and the reaction was conducted for 20 hours at 40°C. The consumption rate of fatty acids was 28% and the glycerides synthesized consisted of 94.4% of monoglyceride, 5.6% of diglycerides, and 0% of triglycerides.

Example 8

1.77 g of vinyl laurate, 8.0 g of glycerol, and 0.55 ml (41.3 units) of a solution containing a lipase from Penicillium cyclopium were mixed and the reaction was conducted for 20 hours at 40°C. The consumption rate of vinyl laurate was 94% and the glycerides synthesized consisted of 96.2% of monoglyceride, 3.8% of diglyceride and 0% of triglyceride.

Example 9

1.77 g of vinyl caprate, 8.0 g of glycerol, 0.2 ml (5 units) of a solution containing a lipase from Penicillium cyclopium, and 0.35 ml of water were mixed, and the reaction was conducted for 20 hours at 40°C. The consumption rate of vinyl caprate was 91 % and the glycerides synthesized consisted of 95.9% of monoglyceride, 4.1 % of diglyceride and 0% of triglyceride.

Example 10

1.77 g of vinyl palmitate or 1.95 g of vinyl stearate, 8.0 g of glycerol, 0.2 ml (13.8 units) of a solution containing a lipase from Penicillium cyclopium, and 0.35 ml of water were mixed and the reaction was conducted for each mixture for 5 hours at 40°C. For each reaction, the consumption rate of fatty acid ester and the composition of glycerides synthesized are shown in Table 6.

TABLE 6

| Raw material fatty acid ester | Consumption rate of fatty acid ester (%) | Mono-glyceride (%) | Di-glyceride (%) | Tri-glyceride (%) |
|---|---|---|---|---|
| Vinyl palmitate | 75 | 94.2 | 5.8 | 0 |
| Vinyl stearate | 47 | 94.6 | 5.4 | 0 |

## Claims

1. A process for producing monoglycerides and diglycerides substantially free from triglycerides which comprises reacting glycerol with a fatty acid or an ester of a fatty acid and agitating the reaction mixture in the presence of a monoglyceride lipase and/or a diglyceride lipase, characterised in that the lipase is enzyme-II derived from Penicillium cyclopium.

2. A process according to claim 1, wherein the reaction is carried out for 1 hour to 5 days with agitation.

3. A process according to claim 1 or claim 2, wherein the lipase is used in an amount of 2 to 10,000 units per mole raw material fatty acid or ester thereof.

4. A process according to any preceding claim, wherein the mole ratio of glycerol to raw material fatty acid or ester is from 0.2-200:1.

5. A process for producing monoglycerides substantially free from triglycerides and diglycerides which comprises reacting glycerol with a fatty acid or an ester of a fatty acid and agitating the reaction mixture in the presence of a monoglyceride lipase and/or a diglyceride lipase, characterised in that the lipase is enzyme-II derived from Penicillium cyclopium, and that the reaction is carried out for 1 to 50 hours at an initial glycerol to raw material fatty acid or ester mole ratio from 0.2-50:1 in the presence of from 2 to 5,000 units of the lipase per mole of raw material fatty acid or ester thereof.

6. A process according to preceding claim, wherein the lipases are produced by Penicillium cyclopium ATCC 34613 for specifically hydrolysing monoglycerides and diglycerides.

7. A process according to claim 6, wherein the lipases used are obtained by removing triglyceride hydrolysing lipase by chromatography from a cultivation mixture of Penicillium cyclopium ATCC 34613.

8. A process according to any preceding claim, wherein the reaction temperature is from 20°C to 55°C.

9. A process according to any preceding claim, wherein the water content in the reaction mixture is 30% by weight or less.

10. A process according to any preceding claim, wherein the fatty acid is a saturated or unsaturated fatty acid having from 4 to 22 carbon atoms.

11. A process according to any preceding claim, wherein the fatty acid is a straight chain fatty acid.

12. A process according to any of claims 1 to 11, wherein the fatty acid is a branched chain fatty acid.

13. A process according to any preceding claim, wherein the ester of the fatty acid is selected from methyl, ethyl, propyl, butyl, benzyl, amyl and vinyl esters of saturated and unsaturated fatty acids having from 4 to 22 carbon atoms.

14. A process according to claim 13, wherein the ester of the fatty acid is an ester of a straight chain fatty acid.

15. A process according to claim 13, wherein the ester of the fatty acid is an ester of a branched chain fatty acid.

## Patentansprüche

1. Verfahren zur Herstellung von Monoglyceriden und Diglyceriden, die im wesentlichen frei von Triglyceriden sind, bei dem Glycerin mit einer Fettsäure oder einem Ester einer Fettsäure umgesetzt wird und die Reaktionsmischung in Gegenwart einer Monoglyceridlipase und/oder einer Diglyceridlipase gerührt wird, dadurch gekennzeichnet, daß die Lipase aus Penicillium cyclopium gewonnenes Enzym II ist.

2. Verfahren nach Anspruch 1, worin die Reaktion über 1 Stunde bis 5 Tage unter Rühren durchgeführt wird.

3. Verfahren nach Anspruch 1 oder 2, worin die Lipase in einer Menge von 2 bis 10 000 Einheiten pro Mol Rohmaterial Fettsäure oder Ester davon verwendet wird.

4. Verfahren nach einem der vorhergehenden Ansprüche, worin das Molverhältnis von Glycerin zu Ausgangsmaterial Fettsäure oder Ester 0,2 bis 200:1 beträgt.

5. Verfahren zur Herstellung von Monoglyceriden, die im wesentlichen frei von Triglyceriden und Diglyceriden sind, bei dem Glycerin mit einer Fettsäure oder einem Ester einer Fettsäure umgesetzt wird und die Reaktionsmischung in Gegenwart einer Monoglyceridlipase und/oder einer Diglyceridlipase gerührt wird, dadurch gekennzeichnet, daß die Lipase aus Penicillium cyclopium gewonnenes Enzym II ist, und daß die Reaktion über 1 bis 50 Stunden bei einem Anfangs-Molverhältnis von Glycerin zu Rohmaterial Fettsäure oder Ester von 0,2 bis 50:1 in der Anwesenheit von 2 bis 5 000 Einheiten der Lipase pro Mol Rohmaterial Fettsäure oder Ester durchgeführt wird.

6. Verfahren nach einem der vorhergehenden Ansprüche, worin die Lipasen durch Penicillium cyclopium ATCC 34613 zum spezifischen Hydrolysieren von Monoglyceriden und Diglyceriden hergestellt werden.

7. Verfahren nach Anspruch 6, worin die verwendeten Lipasen durch Entfernen von triglyceridhydrolysierender Lipase durch Chromatographie aus einer Kulturmischung von Penicillium cyclopium ATCC 34613 erhalten werden.

8. Verfahren nach einem der vorhergehenden Ansprüche, worin die Reaktionstemperatur 20 bis 55 ° C beträgt.

9. Verfahren nach einem der vorhergehenden Ansprüche, worin der Wassergehalt in der Reaktionsmischung 30 Gew.-% oder weniger beträgt.

10. Verfahren nach einem der vorhergehenden Ansprüche, worin die Fettsäure eine gesättigte oder ungesättigte Fettsäure mit 4 bis 22 Kohlenstoffatomen ist.

11. Verfahren nach einem der vorhergehenden Ansprüche, worin die Fettsäure eine geradkettige Fettsäure ist.

12. Verfahren nach einem der Ansprüche 1 bis 10, worin die Fettsäure eine verzweigtkettige Fettsäure ist.

13. Verfahren nach einem der vorhergehenden Ansprüche, worin der Ester der Fettsäure aus Methyl-, Ethyl-, Propyl-, Butyl-, Benzyl-, Amyl- und Vinylestern von gesättigten und ungesättigten Fettsäuren mit 4 bis 22 Kohlenstoffatomen gewählt wird.

14. Verfahren nach Anspruch 13, worin der Ester der Fettsäure ein Ester einer geradkettigen Fettsäure ist.

15. Verfahren nach Anspruch 13, worin der Ester der Fettsäure ein Ester einer verzweigtkettigen Fettsäure ist.

## Revendications

1. Procédé de production de monoglycérides et de diglycérides principalement dépourvus de triglycérides, qui consiste à faire réagir du glycérol avec un acide gras ou un ester d'un acide gras et à agiter le mélange réactionnel en présence d'une monoglycéride-lipase et/ou d'une diglycéride-lipase, caractérisé en ce que la lipase est l'enzyme-II dérivé de Penicillium cyclopium.

2. Procédé suivant la revendication 1, dans lequel la réaction est conduite pendant une durée de 1 heure à 5 jours sous agitation.

3. Procédé suivant la revendication 1 ou la revendication 2, dans lequel la lipase est utilisée en une quantité de 2 à 10 000 unités par mole d'acide gras ou d'ester d'acide gras utilisé comme matière de départ.

4. Procédé suivant l'une quelconque des revendications précédentes, dans lequel le rapport molaire du glycérol à l'acide gras ou à l'ester d'acide gras utilisé comme matière de départ a une valeur de 0,2-200:1.

5. Procédé de production de monoglycérides pratiquement dépourvus de triglycérides et de diglycérides, qui consiste à faire réagir du glycérol avec un acide gras ou un ester d'un acide gras et à agiter le mélange réactionnel en présence d'une monoglycéride-lipase et/ou d'une diglycéride-lipase, caractérisé en ce que la lipase est l'enzyme-II dérivé de Penicillium cyclopium, et en ce que la réaction est conduite pendant 1 à 50 heures à un rapport molaire initial du glycérol à l'acide gras ou à l'ester d'acide gras utilisé comme matière de départ de 0,2-50:1 en présence de 2 à 5000 unités de la lipase par mole d'acide gras ou d'ester d'acide gras utilisé comme matière de départ.

6. Procédé suivant la revendication précédente, dans lequel les lipases sont produites par Penicillium cyclopium ATCC 34613 pour l'hydrolyse spécifique de monoglycérides et de diglycérides.

7. Procédé suivant la revendication 6, dans lequel les lipases utilisées sont obtenues par séparation chromatographique de la lipase hydrolysant les triglycérides d'un mélange de culture de Penicillium cyclopium ATCC 34613.

8. Procédé suivant l'une quelconque des revendications précédentes, dans lequel la température de réaction va de 20 à 55°C.

9. Procédé suivant l'une quelconque des revendications précédentes, dans lequel la teneur en eau du mélange réactionnel est égale ou inférieure à 30 % en poids.

10. Procédé suivant l'une quelconque des revendications précédentes, dans lequel l'acide gras est un acide gras saturé ou non saturé ayant 4 à 22 atomes de carbone.

11. Procédé suivant l'une quelconque des revendications précédentes, dans lequel l'acide gras est un acide gras à chaîne droite.

12. Procédé suivant l'une quelconque des revendications 1 à 11, dans lequel l'acide gras est un acide gras à chaîne ramifiée.

13. Procédé suivant l'une quelconque des revendications précédentes, dans lequel l'ester de l'acide gras est choisi entre les esters de méthyle, d'éthyle, de propyle, de butyle, de benzyle, d'amyle et de vinyle d'acides gras saturés et non saturés ayant 4 à 22 atomes de carbone.

14. Procédé suivant la revendication 13, dans lequel l'ester d'acide gras est un ester d'un acide gras à chaîne droite.

15. Procédé suivant la revendication 13, dans lequel l'ester de l'acide gras est un ester d'un acide gras à chaîne ramifiée.